# EUROPEAN PATENT APPLICATION

(11) **EP 2 020 602 A1**
(43) Date of publication of application: **04.02.2009**
(21) Application number: 07380227.4
(22) Date of filing: 02.08.2007
(51) Int. Cl.: G01N 33/68, C07K 14/47, C07K 16/18

(54) **High sensitivty immunoassays and kits for the determination of peptides and proteins of biological interest**

(71) Applicant: Araclon Biotech, 50004 Zaragoza (ES)
(72) Inventor: Sarasa Barrio, J Manuel, 50005 Zaragoza (ES)
(74) Representative: ABG Patentes, S.L.

(57) **Abstract**

The invention relates to immunoassays which allow the detection of polypeptides in samples with a higher sensitivity than assays of the state of the art. The invention also relates to kits which provide the components needed for carrying out said immunoassays.

## Description

### TECHNICAL FIELD

The invention relates to the field of immunoassays which allow the detection of peptides and proteins in samples and which provide a higher sensitivity than assays of the state of the art. The invention also relates to kits which provide the components needed for carrying out said immunoassays.

### BACKGROUND OF THE INVENTION

Alzheimer's disease (AD) is a progressive degenerative disease of the central nervous system characterized by progressive and increasing memory loss, followed by loss of control of limbs and bodily functions and eventual death. It is by far the most common cause of dementia affecting 1 to 6% of people over the age of 65 years and between 10 to 20% of those over 80.

AD is distinguished from other types of dementia by several pathological features, including the progressive appearance in the brain of the patients of senile plaques in the extracellular space between neurons. The plaques have central cores of amyloid deposits formed mainly by fibrils of a 40-42 amino acids peptide referred to amyloid β peptide (Aβ) surrounded by degenerated neuritis and glial cells. This peptide results from the proteolytic processing of a precursor protein called β amyloid precursor protein (βAPP). βAPP is found in the organism as different isoforms derived from the alternative splicing of the primary transcript of the gene coding for βAPP. These isoforms are mainly βAPP-695, βP-751 and βAPP-770, wherein the figures refer to the number of amino acids. The βAPP gene is found in humans in chromosome 21, whose trisomy (Down's syndrome) results in the overexpression of the protein and the early appearance of amyloid plaques in the brains of patients (Selkoe D.J. (2001) Physiol. Rev. 81, 741-766). βAPP is a transmembrane protein which can be processed by different proteolytic enzymes (secretases) to give rise to different products. Normally, βAPP undergoes cleavage by alpha secretase in the extracellular region of it to generate a long soluble secreted fragment and a shorter membrane-anchored fragment called C83, which is further cleaved by the γ-secretase to produce the p3 peptide (p3₄₀ or p3₄₂) and a 57 or 59 peptide which is further degraded by other proteases. When βAPP is cleaved by β-secretase, it results in a soluble secreted fragment (sβAPP-β) and a C99 fragment which can be cleaved by γ-secretase to give the amyloid peptide Aβ and a 57 or 59 amino acids peptide anchored in the membrane (Selkoe D.J. (2001) Physiol. Rev. 81, 741-766).

AD can be classified according to the age of appearance as early onset (age under 60 years) and late onset (age above 60 years), according to the existence of an autosomic dominant inheritance, as familiar AD or sporadic AD. Early onset familiar forms of AD can be associated to known mutation in the genes coding for βAPP, presenilin 1 and presenilin 2 (located, respectively, on chromosomes 21, 14 and 1). These classifications are not mutually exclusive. The most frequent forms are sporadic late-onset forms.

In clinical praxis, diagnosis of AD is carried out using clinical criteria based on the presence of typical clinical hallmarks and the exclusion of other types of dementia using neuroimaging techniques and blood analysis. Using these criteria, diagnostic reliability is acceptable although, according to studies done using brain autopsy, between 10-20% of the patients diagnosed with AD suffered from a different disease. Moreover, the current diagnostic methods can only be carried out when the neurodegenerative process is so advanced that the patient suffers from severe dementia and the brain damages are so extensive that the number of therapeutic measures is limited. Definitive diagnosis requires pathologic examination of post-mortem brain tissue.

Therefore, there is a need for identifying biomarkers for the early diagnosis of AD which are sensitive and specific and which allow distinguishing cognitive impairment due to age from those associated with the early symptoms of the process, as well as to distinguish changes due to AD and due to other degenerative conditions. According to Growdon et al. (Neurobiol. Aging, 1998, 19:109-116), the ideal marker for AD should meet the following requirements:
- It should detect a fundamental feature of the neuropathology
- It should be validated in neuropathologically-confirmed cases of the disease
- It should show a sensitivity of at least 80% for detecting AD
- It should show a specificity of at least 80% to distinguish AD from other types of dementia and
- It should be reliable, reproducible, non-invasive, simple to perform and inexpensive.

Methods are known in the prior art to diagnose AD by detecting the levels of biomarkers present in the brain or CSF of patients. Different biomarkers have been characterised whose determination is carried out in CSF. CSF reflects directly the composition of the extracellular space of the central nervous system and thus, provides higher concentrations as biomarkers. However, CSF can only be retrieved by means of lumbar punction, which is not a routine diagnostic method easily accepted by patients suffering from dementia, let alone in patients with memory disorders. Thus, there is a need for AD biomarkers which can be detected in samples which can be non-invasively retrieved from the body.

Suitable AD biomarkers described in the prior art and which can be detected in plasma include (i) markers derived from the amyloid plaque, (ii) autoantibodies against Aβ o βAPP, (iii) inflammatory markers such IL-6, its receptor or gp130, C-reactive protein or oxidative stress (isoprostanes), (iv) markers of lipidic metabolism (apoE, oxysterols) and (v) vascular disease markers (homocysteine, lipoprotein b C1q) (Scheuner D et al. (1996) Nature Med 2, 864-870).

However, in view of the fact that Aβ accumulates in the brain of AD patients and is a central element in the pathogenesis of AD, this protein has been considered as the most suitable candidate as AD biomarker. However, the use of Aβ as plasma biomarker for AD faces the problem that the concentrations of the Aβ peptides (Aβ(1-40) and Aβ(1-42)) in serum are extremely low, so that there are no assays which are sensitive enough so as to allow reliable detection of said peptide species.
Scheuner et al (Nature Med., 1996, 2:864-870) performed a preliminary study to detect whether extracellular concentrations of presenilin 1, presenilin 2 or βAPP were increased in patients from families carrying mutations associated to familiar AD. Although elevated levels of Aβ 1-42(43) was observed in the plasma from subjects with FAD-linked PS1 (P < 0.0001), PS2_{N141I} (P = 0.009), APP_{K670N}, _{M671L} (P < 0.0001), and APP_{V7171} (one subject) mutations, these studies were not validated for analysing sporadic AD, wherein the concentrations of Aβ peptides in serum is much lower. The assay kit used in this document is the ELISA sandwich assay previously described by Suzuki,N. et al. (Science, 1994, 264:1336-1340), which uses a capture antibody and peroxidase-conjugated detection antibody.

It has been suggested that there is no correlation between Aβ levels and sporadic AD. Accordingly, Tamaoka A et al. (J Neurol Sci., 1996, 141, 65-68) measured Aβ levels in plasma from 28 patients with sporadic AD, 40 patients with different neurological process with dementia and 40 healthy controls. The authors concluded that the plasma levels of Aβ40 and Aβ42 are similar in control subjects and patients suffering from sporadic AD. Similar results were obtained by Kosaka et al. (Neurology, (1997) 48, 741-745). The assay used by Tamaoka and co-workers has been further characterised in WO200722015 for the measurement of Aβ1-40 in plasma. The assay is an ELISA sandwich assay wherein the Aβ40 and Aβ42 peptides are captured on a solid support using the 1A10 monoclonal antibody (mAb) and detected using peroxidase-conjugated 14F1 mAb. This assay provides a sensitivity level in the range of single digit pg/ml.

Vanderstichele H et al. (Amyloid, 2000, 7, 245-258) determined the levels of Aβ(1-42) in CSF, plasma and urine in 12 healthy controls, 39 AD patients and 6 patients suffering from Lewy body dementia, 10 patients with other types of dementia and 9 patients with other neurological pathologies showing no dementia, but no correlation could be observed between Aβ(1-42) levels and diagnostic, sex or MMSE results. For this studies, the INNOTEST β-amyloid(1-42) test was used based on a ELISA sandwich assay wherein the Aβ(1-42) is captured on a solid support using the 21F12 mAb which recognises the N-terminal region of Aβ(1-42) and detected using biotinylated 3D6 mAb which recognises the C-terminal region of Aβ(42) and streptavidin coupled to HRP.

Fukomoto y col. (Arch. Neurol. 2003, 60, 958-964) studied the correlation of plasma Aβ40 and Aβ42 levels with respect to different clinical, demographic and genetic variables in 146 AD patients, 37 patients with mild cognitive impairment and 96 patients with Parkinson disease. The results indicated that there is a significant increase with age but no differences with respect to diagnosis, family history, ApoE genotype or treatment with different drugs such as acetyl cholinesterase inhibitor or statins. The measurements were done using an ELISA sandwich assay using a mAb which recognises amino acids 11 to 28 of Aβ (BNT77) and HRP-coupled mAbs which specifically recognise Aβ40 and Aβ42 (BA27 and BC05 mAbs).

Mehta et al. (Arch. Neurol. 57, 2000, 100-105) measured plasma and CSF Aβ40 and Aβ42 levels in 78 AD patients and 61 healthy controls and observed that the Aβ40 plasma levels are higher in AD patients with an ApoE4 allele than in the control group without said allele, that the Aβ42 levels are similar between AD patients and controls and no differences with respect to sex o mini mental state examination (MMSE) score. The measurements were done using an ELISA sandwich assay which used the anti-Aβ mAb 6E10 as capture antibody and a biotinylated antiserum raised against peptides specific for Aβ42 and Aβ40.

Mayeux, R. et al. (Ann Neurol. 1999, 46, 412-416) measured the plasma Aβ40 and Aβ42 levels in a cohort of subjects (530) and 18 months later (307 subjects) using he same ELISA assay as Mehta et al. (Arch. Neurol. 57, 2000, 100-105). They observed that the subjects which developed AD had higher Aβ42 levels than those that did not develop AD during the study. Aβ42 levels decreased after three years of established AD diagnosis. No differences were observed with respect to AD phenotype.

Lanz, T.A and Schacthter, J.B. (J. Neuroscience Methods, 2006, 157:71-81) describe a colorimetric and fluorometric ELISA sandwich assays for detecting either the total content of Aβ peptides or the individual amounts of Aβ40 and Aβ42 peptides. For detecting the total amount of Aβ peptides, the 6E10 mAb is used to capture the Aβ peptides and then detected using biotinylated 4G8 and either Europium-conjugated streptavidin or HRP-streptavidin. For detecting the amount of Aβ40 and Aβ42 peptides, the 6E10 mAb is used as capture antibody and biotinylated rabbit polyclonal antibodies specific for each of the fragments followed by either Europium-conjugated streptavidin or HRP-streptavidin. However, this method provided a lowest detection limit of 10 pg/ml and was only used to measure Aβ in brain extracts.

WO200750359 describes an immunoassay for detecting multimeric forms of Aβ42 which involves capturing the Aβ42 derived peptides using polyclonal antibodies specific for said multimeric forms and detecting the amount of peptides bound using a mAb and HRP-conjugated anti-mouse IgG. However, this assay provides a detection limit of 1000-3000 pM, corresponding to 4000 pg/ml.

WO0162801 describes an ELISA sandwich assay for determining Aβ which uses the 3D6 mAb anti-N-terminal as coating antibody and a mAb which recognises amino acids 15 to 24 of mature Aβ for detection, but is only used for measuring Aβ concentration values in the range of 100 to 200 pg/mL.

WO0315617 describes an ELISA assay for measuring Aβ40 and Aβ42 in plasma which uses an antibody which recognises amino acids 13 to 28 of the mature Aβ peptides but is only capable of detecting concentrations of said peptides in the range of 250-400 pg/ml and thus, only suitable for measuring Aβ in plasma with a familiar AD.

WO0246237 describes an ELISA sandwich assay for measuring total Aβ species (Aβ40 and Aβ42) in brain homogenate which uses an mAb specific for amino acids 13 to 28 of Aβ42 as capture antibody and biotinylated 3D6 mAb specific for the N-terminal region of the Aβ42 peptide followed by streptavidin conjugated to peroxidase. This document also describes a Aβ42 specific ELISA sandwich assay which uses mAb 21F12 specific for amino acids 33-42 of Aβ42 as capture antibody and biotinylated 3D6 mAb as detection antibody. However, these assays have lower sensitivity levels of 50 ng/ml for the total Aβ assay and 125 mg/ml for the Aβ42-specific assay, which make them unsuitable for measuring total Aβ or Aβ42 in plasma or serum.

WO0413172 describes an ELISA sandwich assay for measuring β-amyloid(1-42) in formic acid brain extracts and CSF using mAb 3D6 as detection antibody and polyclonal antibodies specific for different regions of the mature Aβ peptides as capture antibody.

However, all the ELISA-based assays known to date have a lower detection limit which is in the range of single digit pg/mL at the most, which is sufficient for detecting Aβ40 and Aβ42 in CSF as well as for detecting said species in plasma in patients suffering from familiar AD, but are unsuitable for detecting Aβ42 in the plasma of patients suffering from sporadic AD, wherein the Aβ42 plasma concentration are much lower. In particular, the INNOTEST β amyloid (1-42) test has a lower detection limit of 20 pg/ml, which allows the detection of Aβ42 in CSF as well as the plasma measurement in patients with familiar AD, but are no sensitive enough for detecting Aβ42 in patients suffering from sporadic AD which show much lower levels of Aβ42 in plasma. This kit is only recommended for measuring Aβ peptide levels in CSF. This recommendation has been further validated by a recent study using the INNOTEST β-amyloid(1-42) wherein it has been shown that the peptide levels in CSF are between 100 and 1000 times higher than in plasma (Lewczuk, P et al. (2004) Neurobiol. Aging 25, 273-281).

Another commercial kits are the Biosource β amyloid (1-40) and β amyloid (1-42) kits. These kits provide an ELISA sandwich assay wherein the peptides are captured on the support using a mAb directed against the amino terminus of mature peptide and detected using a rabbit polyclonal antibody which reacts with the C-terminal region of the mature β-peptides and anti-rabbit IgG peroxidase. This kit has sensitivity, according to the manufacturer of less than 10 pg/mL, but is used to detect Aβ concentrations between 15.6 and 1000 pg/mL. The Biosource kits are recommended by the manufacturers for detection of Aβ peptides in serum, CSF and cell culture. However, average Aβ40 and Aβ42 levels in serum in sporadic AD patients are below the lower sensitivity limit of the assays so that it does not seem possible to use them for measurement in serum.

Canadian patent application (CA2585148), which corresponds to the international patent application WO200646644, describes the only Aβ peptide assay showing a lower detection limit of 0.5 pg/mL. The assay used in this document is an electrochemiluminiscent (ECL) sandwich assay wherein the mAb 21F12 (which recognises amino acids 33-42 of Aβ42) is coupled to magnetic beads, which are then used to capture the Aβ42 peptide in the sample containing Aβ42 and further contacted with 3D6 mAb coupled to a ruthenium complex. The amount of 3D6 antibody bound is then detected by the luminescence emitted by the ruthenium complex when electrical energy is applied. Using this assay, the inventors are capable of detecting as low as 0.5 pg/mL of a Aβ42 standard. However, when the same assay is used to compare Aβ42 in plasma samples from AD patients and healthy controls, no significant differences could be observed between the two sets of patients, which led the inventors to conclude that the amount of intact Aβ42 in serum is very low due to degradation and turned to a competitive ELISA assay using 21F12 mAb which provides lower sensitivity levels in the range of ng/mL.

Therefore, there is a need in the art for improved immunological assays and kits to detect Aβ-derived peptides which overcome the problems of the methods and kits known in the art, in particular, which are sensitive enough to detect Aβ peptides in a reliable manner in plasma of patients suffering from sporadic AD.

### SUMMARY OF THE INVENTION

In a first aspect, the invention relates to a kit for the detection or determination of a target polypeptide comprising
(i) a first antibody or combination of antibodies which recognise said target polypeptide
(ii) a second antibody or combination of antibodies which recognise a different region of the target polypeptide than the region recognised by the first antibody or combination of antibodies
(iii) a reagent showing affinity for the second antibody which is coupled to a first member of a binding pair and
(iv) a second member of a binding pair coupled to a detectable tag.

In a second aspect, the invention relates to the use of the kit of the invention to detect or determine a Aβ40 and/or Aβ42 in a sample.

In a third aspect, the invention relates to a method for determining or detecting the amount of a target polypeptide in a sample comprising the steps of
(i) capturing the target polypeptide present in the sample with a first antibody or combination of antibodies which bind specifically said target polypeptide,
(ii) contacting the immune complexes formed in step (a) with a second antibody or combination of antibodies which recognise a region of the target polypeptide which is different from the region that is recognised by the first antibody or combination of antibodies,
(iii) contacting the complexes formed in step (ii) with a reagent which shows affinity for the second antibody and which is coupled to a first member of a binding pair,
(iv) contacting the complexes formed in step (iii) with a second member of a binding pair which is coupled to a detectable tag and
(v) determining or detecting the activity or amount of detectable tag attached to the second member of the binding pair.

In a fourth aspect, the invention relates to a method for the diagnosis of a degenerative disorder in a subject which comprises determining the amount of Aβ40 or Aβ42 in a sample of a patient using a method according to the invention and correlating the concentration of one or both peptides in the sample of said subject with respect to the concentration of said peptide or peptides in a sample from a healthy individual with the appearance of the degenerative disorder.

### BRIEF DESCRIPTION OF THE FIGURES

Figure 1 shows the standard titration curve of the ELISA sandwich assay wherein the absorbance values at 450 nm are plotted against known concentrations of standard preparations of Aβ40 (in pg/mL).
Figure 2 shows the standard titration curve of the ELISA sandwich assay wherein the absorbance values at 450 nm are plotted against known concentrations of standard preparations of Aβ42 (in pg/mL).

### DETAILED DESCRIPTION OF THE INVENTION

The authors of the present invention have surprisingly found that by using a kit having the components as defined in claim 1, it is possible to determine the levels of Aβ40 and Aβ42 with a lower detection limit of less than 0.1 pg/ml. This kit allows a reliable quantification of said molecular species in any sample in any subject and, in particular, in the plasma from subjects suspected of suffering sporadic AD, wherein the plasma concentrations are so low that no reliable measurement has been possible up to date. Moreover, the improvement which has been achieved using the kit of the invention need not be restricted to the detection of Aβ4 peptides but can be equally applicable to increase the sensitivity of any immunodetection method known to date so as to allow the detection of said target substances in samples wherein their concentrations are found below the sensitivity levels of the assays known to date.

Accordingly, in a first aspect, the invention relates to a kit for the determination or the detection of a target polypeptide comprising
(i) a first antibody or combination of antibodies which recognise said target polypeptide
(ii) a second antibody or combination of antibodies which recognise a different region of the target polypeptide than the region recognised by the first antibody or combination of antibodies
(iii) a reagent showing affinity for the second antibody which is coupled to a first member of a binding pair and
(iv) a second member of a binding pair coupled to a detectable tag.

Without wishing to be bound by any particular theory, it is believed that the enhanced sensitivity is due to the use of the reagent (iii) which allows the signal resulting from the detection antibody to be amplified.

A polypeptide, as it is understood herein, comprises any molecule, natural or synthetic, comprising a chain of amino acids of any length linked together by peptidic bonds and whose detection in a sample might be of relevance for basic research, diagnostic purposes, environmental studies, and the like. However, in a preferred embodiment, the first antibody specifically recognises Aβ40 and/or Aβ42 peptides.

In a still more preferred embodiment, the kit of the invention is used for detecting a target peptide which is selected from the group of Aβ42, Aβ40 or the combination of both and both the first antibody or combination of antibodies and the second antibody or combination of antibodies recognise Aβ42 and/or Aβ40.

"Aβ42", as used herein, relates to a 42 amino acids peptide corresponding to amino acids 672 to 713 (SEQ ID NO:4) and which is produced by the sequential proteolytic cleavage of the amyloid precursor protein (SEQ ID NO:6) by the β- and γ-secretases.

"Aβ40", as used herein, relates to a 40 amino acids peptide corresponding to amino acids 672 to 711 (SEQ ID NO:5) and which is produced by the sequential proteolytic cleavage of the amyloid precursor protein (SEQ ID NO:6) by the β- and γ-secretases.

In the context of the present invention, the first antibody will be referred to as "capture antibody", meaning that this antibody is used to retrieve from a sample all molecular species to which the antibody specifically binds. There is practically no limitation with regard to the type of antibody that can be used as capture antibody as long as it contains at least one antigen binding site specific for Aβ40 and/or Aβ42. Therefore, antibodies molecules suitable for use as capture antibodies include
- "intact" antibodies which comprise an antigen-binding variable region as well as a light chain constant domain (CL) and heavy chain constant domains, CH1, CH2 and CH3,
- "Fab" fragments resulting from the papain digestion of an intact antibody and which comprise a single antigen-binding site and a CL and a CH1 region,
- "F(ab')₂" fragments resulting from pepsin digestion of an intact antibody and which contain two antigen-binding sites,
- "Fab'" fragments contain the constant domain of the light chain and the first constant domain (CH1) of the heavy chain and has one antigen-binding site only. Fab' fragments differ from Fab fragments by the addition of a few residues at the carboxy terminus of the heavy chain CH 1 domain including one or more cysteines from the antibody hinge region.
- "Fv" is the minimum antibody fragment which contains a complete antigen-recognition and antigen-binding site. This region consists of a dimer of one heavy chain and one light chain variable domain in tight, non-covalent-association. It is in this configuration that the three hypervariable regions (CDRs) of each variable domain interact to define an antigen-binding site on the surface of the VH - VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.
- Single-chain FV or "scFv" antibody fragments comprise the VL and VH, domains of antibody, wherein these domains are present in a single polypeptide chain. Preferably, the VL and VH regions are connected by a polypeptide linker which enables the scFv to form the desired structure for antigen binding.
- "Diabodies" comprise a heavy chain variable domain (VH) connected to a light chain variable domain (VL) on the same polypeptide chain (VH-VL) connected by a peptide linker that is too short to allow pairing between the two domains on the same chain. This forces pairing with the complementary domains of another chain and promotes the assembly of a dimeric molecule with two functional antigen binding sites.
- "Bispecific antibodies" (BAbs) are single, divalent antibodies (or immunotherapeutically effective fragments thereof) which have two differently specific antigen binding sites. The two antigen sites may be coupled together chemically or by genetic engineering methods known in the art.

All these antibody fragments can be further modified using conventional techniques known in the art, for example, by using amino acid deletion(s), insertion(s), substitution(s), addition(s), and/or recombination (and/or any other modification(s) (e.g. posttranslational and chemical modifications, such as glycosylation and phosphorylation) known in the art either alone or in combination. Methods for introducing such modifications in the DNA sequence underlying the amino acid sequence of an immunoglobulin chain are well known to the person skilled in the art; see, e.g., Sambrook et al.; Molecular Cloning: A Laboratory Manual; Cold Spring Harbor Laboratory Press, 2nd edition 1989 and 3rd edition 2001.

Antibodies suitable as capture antibodies include both polyclonal and monoclonal antibodies. For the production of polyclonal antibodies, various hosts including goats, rabbits, rats, mice, camels, dromedaries, llamas, humans, birds and others may be immunized by injection with a peptide corresponding to a fragment of Aβ40 or Aβ42 which has immunogenic properties. Depending on the host species, various adjuvants may be used to increase immunological response. Such adjuvants include, but are not limited to, Freund's, mineral gels such as aluminium hydroxide, and surface active substances such as lysolecithin, polyanions, peptides, oil emulsions, KLH, and dinitrophenol. Among adjuvants used in humans, BCG (bacilli Calmette-Guerin) and *Corynebacterium parvum* are especially preferable. If the antigen is a peptide, it may be useful to conjugate it to a protein that is immunogenic in the species to be immunized. For example, the antigen can be conjugated to keyhole limpet hemocyanin (KLH), Blue Carrier (hemocyanin isolated from *Concholepas concholepas*), bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride or SOCl₂.

For the production of monoclonal antibodies, conventional techniques can be used. For instance, monoclonal antibodies may be made using the hybridoma method first described by Kohler et al., Nature, 256:495 (1975) using the procedure described in detail in units 11.4 to 11.11 of Ausubel, F.M. et al. (Current Protocols in Molecular Biology, John Wiley & Sons Inc; ring-bound edition, 2003). Alternatively, monoclonal antibodies can be isolated by recombinant DNA procedures from antibody phage libraries generated using the techniques described in McCafferty et al., Nature, 348:552-554 (1990). Clacksoii et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nucl. Acids. Res., 21:2265-2266 (1993)). Thus, these techniques are viable alternatives to traditional monoclonal antibody hybridoma techniques for isolation of monoclonal antibodies.

Polyclonal antibodies can be used directly as an antiserum obtained from immunised hosts after bleeding and removal of the fibrin clot. Monoclonal antibodies can be used directly as the supernatant of the hybridoma culture or as ascites fluid after implantation of the hybridoma in the peritoneal cavity of a suitable host. Alternatively, the immunoglobulin molecules, either polyclonal or monoclonal, can be purified prior to their use by conventional means such as affinity purification using peptides derived from Aβ40 or Aβ42, non-denaturing gel purification, HPLC or RP-HPLC, size exclusion, purification on protein A column, or any combination of these techniques.

In a preferred embodiment, the capture antibodies recognise a region common to Aβ40 and Aβ42, so as to allow simultaneous capture of both species with a single antibody species. In principle, any antibody specific for a region common to the sequences of both Aβ40 and Aβ42 can be used as capture antibody. Preferred epitopes which can be targeted by the capture antibody include epitopes located within amino acids 1 to 16, 1 to 17, 13 to 28, 15 to 24, 1 to 5 and 1 to 11 of Aβ40 or Aβ42. In a more preferred embodiment, the capture antibody is directed against a region in Aβ40 and/or Aβ42 which is different from the C-terminal region. In a still more preferred embodiment, the capture antibody is directed against an epitope of the N-terminal region of the Aβ40 and Aβ42 peptides. In yet another preferred embodiment, the capture antibody is a monoclonal antibody. In a still more preferred embodiment, the capture antibody recognises a region corresponding to amino acids 1-16 of the Aβ peptides. In a still more preferred embodiment, the monoclonal antibody used as capture antibody is the 6E10 mAb as has been described in Kim, K.S. (Neuroscience Res. Comm. 1988, 2:121-130).

The second component of the kit of the invention corresponds to an antibody or combination of antibodies which recognise a different region of the target polypeptide than the region recognised by the first antibody or combination of antibodies. In the context of the present invention, the second antibody will be referred to as "detection antibody", since this antibody will be used to detect the amount of antigen which has been retained by the capture antibody.

As with the capture antibody, there is practically no limitation with regard to the type of antibody that can be used as detection antibody. However, it will be also understood by the person skilled in the art that the detection antibody (i) must bind to a region of the antigen which is not covered by the capture antibody and (ii) must contain not only the antigen binding site but also an additional region or regions that can be specifically detected by a reagent showing high affinity binding for said antibody, so as to allow detection of the antibody which is bound to the antigen captured by the capture antibody. Preferably, said additional regions which can be specifically bound by said reagent correspond to the constant region of the immunoglobulin molecule.

Suitable detection antibodies include intact antibodies, Fab, F(ab')₂, Fab' and Fv fragments, single-chain FV antibodies, diabodies, bispecific antibodies and the like, wherein these compounds are as defined previously. Similarly as with the case of the capture antibody, the detection antibody can be polyclonal or monoclonal, native or post-translationally modified and pure or enriched in the antigen-binding molecules, using the same procedures as described for the capture antibodies. It will be appreciated by the skilled person that, in order to use the detection antibody, it must be diluted to a suitable working concentration and that said dilution can be routinely determined for each batch of antibody. Moreover, it will be evident that the adequate working dilution will differ depending on whether the antisera is used or rather a purified IgG fraction. Typical dilutions are 1/1000, 1/2000, 1/3000, 1/4000, 1/5000, 1/6000, 1/7000, 1/8000, 1/9000, 1/10000 and the like.

In a preferred embodiment, the detection antibodies comprises any polyclonal antibody selected from the group of
(i) a polyclonal antibody prepared against a peptide corresponding to the C-terminal region of the Aβ42 peptide which binds specifically to Aβ42 without giving any substantial cross-reaction with Aβ40 or Aβ43
(ii) a polyclonal antibody prepared against a peptide corresponding to the C-terminal region of the Aβ40 peptide which binds specifically to Aβ40 without giving any substantial cross-reaction with Aβ42, Aβ39, Aβ38, Aβ41 or Aβ43
(iii) an antibody that recognises simultaneously the C-terminal region of both Aβ40 and Aβ42 or
(iv) a combination of the antibodies under (i) and (ii).

In a more preferred embodiment, the peptide corresponding to the C-terminal region of the Aβ42 peptide used to prepare the Aβ42-specific antibody is a peptide as defined in SEQ ID NO:1 or SEQ ID NO:2. In another preferred embodiment, the peptide corresponding to the C-terminal region of the Aβ40 peptide used to prepare the Aβ40-specific antibody is a peptide as defined in SEQ ID NO:3. Antibodies specific for Aβ40 and Aβ42 and methods for their preparation have been described in detail in WO2004024770 and WO2004098631, whose contents are incorporated herein by reference.

It will be understood to a person of ordinary skilled in the art that the method can be used for detecting Aβ40, Aβ42 or both species simultaneously depending on the type of capture and detection antibodies used in the method.

In order to detect or determine specifically Aβ40, the capture antibody can be an antibody which recognises the N-terminal region of Aβ40 (and also of Aβ42, since both peptides have identical N-terminal regions) and the detection antibody can be an antibody which recognises specifically the C-terminal region of Aβ40 without giving any cross-reaction with Aβ42. Alternatively, Aβ40 can be specifically detected using a capture antibody which recognises the C-terminal region of Aβ40 without giving any cross-reaction with Aβ42 and a detection antibody which recognises a region of Aβ40 which is common to both Aβ40 and Aβ42, preferably the N-terminal region of Aβ42/Aβ40.

In order to detect or determine specifically Aβ42, the capture antibody can be an antibody which recognises the N-terminal region of Aβ42 (and also of Aβ40, since both peptides have identical N-terminal regions) and the detection antibody can be an antibody which recognises specifically the C-terminal region of Aβ42 without giving any cross-reaction with Aβ40. Alternatively, Aβ42 can be specifically detected using a capture antibody which recognises the C-terminal region of Aβ42 and a detection antibody which recognises a region of Aβ42 which is common to both Aβ42 and Aβ40.

In order to detect or determine simultaneously Aβ42 and Aβ40, the capture antibody can be an antibody which recognises the N-terminal region common to Aβ42 and Aβ40 and the detection antibody can be a combination of at least two antibodies, wherein the first antibody recognises specifically the C-terminal region of Aβ42 without giving any cross-reaction with Aβ40 and the second antibody recognises specifically the C-terminal region of Aβ40 without giving any cross-reaction with Aβ42. Alternatively, capture antibody can be an antibody which recognises the N-terminal region common to Aβ42 and Aβ40 and the detection antibody can be an antibody that recognises the C-terminal region of both Aβ40 and Aβ42. Alternatively, Aβ42 and Aβ40 can be simultaneously detected using as capture antibody a mixture of at least two antibodies comprising a first antibody which recognises specifically the C-terminal region of Aβ42 without giving any cross-reaction with Aβ40 and a second antibody which recognises specifically the C-terminal region of Aβ40 without giving any cross-reaction with Aβ42 and a detection antibody which recognises the N-terminal region common to both Aβ42 and Aβ40. Alternatively, Aβ42 and Aβ40 can be simultaneously detected using as capture antibody an antibody which recognises the C-terminal region of both and Aβ42 and a detection antibody which recognises the N-terminal region common to both Aβ42 and Aβ40.

The third element of the kit corresponds to a reagent which shows affinity for the detection antibody and which is coupled to a first member of a binding pair. The antibody-binding reagent may non-covalently bind to a particular type(s), a particular class(es) and/or a particular subclass(es) of an antibody or antibody fragments. Alternatively, the antibody- binding reagent may non-covalently bind to an antibody specific for a particular antigen. In certain embodiments, the antibody-binding reagent binds non-covalently to the Fc region or to the F(ab) region of the detection antibody. Preferred antibody-binding reagents include protein A, protein G, protein V, protein L, an anti-Fc antibody or antibody-binding fragment and an Fc receptor (FcR) or an antibody-binding fragment thereof. Non-limiting examples of antibodies which can be non-covalently bound to the detection antibody include monoclonal antibodies, polyclonal antibodies, multispecific antibodies, human antibodies, humanized antibodies, chimeric antibodies, single domain antibodies, single chain Fvs (scFv) single chain antibodies, Fab fragments, F(ab') fragments, disulfide-linked Fvs (sdFv), intrabodies, and anti-idiotypic (anti- Id) antibodies, and epitope-binding fragments of any of the above. Non-limiting examples of Fc receptors include FcγRI, FcγRIIA, FcγRIIB, FcγRIIC, FcγRIIIAα, FcγRIIIB, FcεRIα, FcεRIξ and FcγRIIIAξ.

The fourth element of the kit of the invention corresponds to a second member of a binding pair which is coupled to a detectable tag. Suitable binding pairs include
■ hapten or antigen/antibody, e.g. digoxin and anti-digoxin antibodies
■ biotin or biotin analogues (e.g. aminobiotin, iminobiotin or desthiobiotin)/avidin or streptavidin,
■ sugar/lectin,
■ enzyme and cofactor
■ folic acid/folate
■ double stranded oligonucleotides that selectively bind to proteins/, transcription factors.
■ nucleic acid or nucleic acid analogue/complementary nucleic acid,
■ receptor/ligand, e.g., steroid hormone receptor/steroid hormone.

It will be understood that the term "first" and "second" member of a binding pair is relative and that each of the above members can be seen as first or second members of the binding pair. In a preferred embodiment, the first member of a binding pair is biotin or a functionally equivalent variant thereof and the second member of the binding pair is avidin, streptavidin or a functionally equivalent variant thereof.

In a preferred embodiment, the second member of the binding pair is streptavidin.

Suitable detectable tags include, without limitation, fluorescent moieties (e.g., fluorescein, rhodamine, phycoerythrin, coumarin, oxazine, resorufin, cyanine and derivatives thereof.), luminescent moieties (e.g., Qdot™ nanoparticles supplied by the Quantum Dot Corporation, Palo Alto, CA). If the detectable tag is an enzyme, then this enzyme must be capable of generating a detectable signal, for example, upon addition of an activator, substrate, amplifying agent and the like. Enzymes which are suitable as detectable tags for the present invention and the corresponding substrates include:
- Alkaline phosphatase:
   ○ Chromogenic substrates: Substrats based on p-nitrophenyl phosphate (p-NPP), 5-bromo-4-chloro-3-indolyl phosphate/nitroblue tetrazolium (BCIP/NPT), Fast-Red/naphthol-AS-TS phosphate
   ○ Fluorogenic substrates: 4-methylumbelliferyl phosphate (4-MUP), 2-(5'-chloro-2'-phosphoryloxyphenyl)-6-chloro-4-(3H)-quinazolinone (CPPCQ), 3,6-fluorescein diphosphate (3,6-FDP), Fast Blue BB, Fast Red TR, or Fast Red Violet LB diazonium salts
- Peroxidases:
   ○ Chromogenic substrates based on 2,2-azinobis(3-ethylbenzothiazoline-6-sulfonic acid) (ABTS), o-phenylenediamine (OPT), 3,3',5,5'-tetramethylbenzidine (TMB), o-dianisidine, 5-aminosalicylic acid, 3-dimethylaminobenzoic acid (DMAB) and 3-methyl-2-benzothiazolinehydrazone (MBTH), 3-amino-9-ethylcarbazole (AEC)- and 3,3'-diaminobenzidine tetrahydrochloride (DAB).
   ○ Fluorogenic susbtrates: 4-hydroxy-3-methoxyphenylacetic acid, reduced phenoxazines and reduced benzothiazines, including Amplex^{®} Red reagent, Amplex UltraRed and reduced dihydroxanthenes.
- Glycosidases:
   ○ Chromogenic substrates: o-nitrophenyl-β-D-galactoside (o-NPG), p-nitrophenyl-β-D-galactoside and 4- methylumbelliphenyl-β-D-galactoside (MUG) for β-D-galactosidase.
   ○ Fluorogenic substrates: resorufin β-D-galactopyranoside, fluorescein digalactoside (FDG), fluorescein diglucuronide, 4-methylumbelliferyl β-D-galactopyranoside, carboxyumbelliferyl β-D-galactopyranoside and fluorinated coumarin β-D- galactopyranosides.
- Oxidoreductases (luciferase):
   ○ Luminiscent substrates: luciferin.

In a preferred embodiment, the detectable tag is horseradish peroxidase and the detection reagent is TMB.

In a preferred embodiment, the kit further comprises a solid support. As used herein, the term "support" or "surface" refers to a solid phase which is a porous or non-porous water insoluble material that can have any one of a number of shapes, such as strip, rod, particles, including latex particles, magnetic particles, microparticles, beads, membranes, microtiter wells and plastic tubes. In principle, any material is suitable as solid support provided that is able to bind sufficient amounts of the capturing antibody. Thus, the choice of solid phase material is determined based upon desired assay format performance characteristics. Materials suitable for the solid support include polymeric materials, particularly cellulosic materials and materials derived from cellulose, such as fibre containing papers, e.g., filter paper, chromatographic paper, glass fiber paper, etc.; synthetic or modified naturally occurring polymers, such as nitrocellulose, cellulose acetate, poly (vinyl chloride), polyacrylamide, cross linked dextrane, agarose, polyacrylate, polyethylene, polypropylene, poly(4-methylbutene), polystyrene, polymethacrylate, poly(ethylene terephthalate), nylon, polyvinyl butyrate), etc.; either used by themselves or in conjunction with other materials; glass such as, e.g., glass available as bioglass, ceramics, metals, and the like. Non-crosslinked polymers of styrene and carboxylated styrene or styrene functionalized with other active groups such as amino, hydroxyl, halo and the like are preferred. In some instances, copolymers of substituted styrenes with dienes such as butadiene will be used.

The solid support and the capture antibody may be separately provided in the kit or, alternatively, the support may be delivered already precoated with the capture antibody. In this case, the support may have been treated with a blocking solution after the binding of the capture antibody. If the support is precoated, it is preferred that the support is treated with a concentrated trehalose solution and allowed to dry, in which case the dry trehalose forms a halo on the support. These supports containing the dry trehalose are exceptionably stable and can be stored for up to two years when kept at 4°C in the dark.

Additional components of the kit may include:
- Means for removing from the patient the sample to be analysed.
- Buffers and solutions required for preparing the standard curves of the target peptides.
- Buffers and solutions for washing and blocking the solid support during the assay
- Buffers and solutions for coating the solid support with the coating antibody
- Reagents for developing the coloured or fluorogenic signal from the detectable tag.
- Reagents for stopping the formation of the coloured or fluorogenic product from the detectable tag (e.g. 1N H₂SO₄)
- Means for maintaining the peptides in an unfolded state (e.g. concentrated guanidinium hydrochloride).
- A sample containing a stock solution of the Aβ40 or Aβ42 peptides or a combination thereof.

In a preferred embodiment, the capture antibody is immobilised onto a solid support. The immobilisation can be carried out prior to the binding of the target polypeptide to be detected or once the peptide/protein is bound to the capture antibody. In any, case, if a solid support is used, it is convenient to block the excess of protein binding sites on the carrier prior to the addition of the sample containing the target polypeptide to be determined. Preferably, blocking or quenching of the peptide-binding sites on the support is carried out using the same buffer which is used for washing the complexes after each binding reaction (e.g. 50 mM Tris-HCl, pH 8, PBS or TBS optionally, comprising Tween 20) supplemented with a macromolecular compound (e.g. bovine serum albumin, non-fat dry milk, western blocking reagent, caseine, lactoalbumine, ovoalbumine) in concentrations from about 0.05% to 10%, preferably 1 to 5%, more preferably around 3%. If the support comprising the immobilised capture antibody must be stored for some time, it is preferred that the support is treated with a concentrated trehalose solution and allowed to dry, in which case the dry trehalose forms a halo on the support. These supports containing the dry trehalose are exceptionably stable and can be stored up to two years when kept at 4°C in the dark.

The kits of the invention allow the detection or determination with high sensitivity of the polypeptides which are specifically recognised by the first and second antibody components of the kit. Thus, in a further aspect, the invention relates to the use of a kit of the invention to detect a protein or protein in a sample. In a preferred embodiment, the kit is used to detect a peptide selected from the group of Aβ40, Aβ42 and the combination thereof in a sample.

A "sample", as understood in the present invention, includes any one of tissue culture, plasma, serum, saliva, semen, sputum, cerebral spinal fluid (CSF), tears, mucus, sweat, milk, brain extracts and the like. In a preferred embodiment, the sample is a plasma sample.

In view of the ability of the kit of the invention to provide high sensitivity determination of the concentrations of Aβ40 and Aβ42 in any sample, it can be used for the diagnosis of any disease wherein there is an altered concentration of any of these two peptides in any cell fluid or tissue, in particular, degenerative diseases and, more particularly, neurodegenerative diseases. Non limitative examples of degenerative diseases which may be diagnosed based on the appearance of altered levels of Aβ40 and/or Aβ42 include:
- bone degenerative disorders such as osteopenia, osteomalacia, osteoporosis, osteomyeloma, osteodystrophy, Paget's disease, osteogenesis imperfecta, bone sclerosis, aplastic bone disorder, humoral hypercalcemic myeloma, multiple myeloma and bone thinning following metastasis.
- cartilage degenerative disorders such as Gorham-Stout syndrome; arthritic diseases; osteoarthritis; rheumatoid arthritis; psoriatic arthritis; rheumatoid disease; and brittle bone disease.
- muscle degenerative diseases such as muscular dystrophy, muscle atrophy, congestive obstructive pulmonary disease, muscle wasting syndrome, sarcopenia, cachexia.
- heart degenerative diseases including cardiac cell death due to ischemia, tissue and organ death due to transplant rejection, hearing loss due to autotoxicity.
- retinal degenerative disorders such as retinitis pigmentosa
- degenerative diseases of the nervous system such as Alexander disease, Alper's disease, Alzheimer's disease, Amyotrophic lateral sclerosis, Ataxia telangiectasia, Batten disease, Bovine spongiform encephalopathy (BSE), Canavan disease, Cockayne syndrome, Corticobasal degeneration, Creutzfeldt-Jakob disease, Huntington disease, HIV-associated dementia, Kennedy's disease, Krabbe disease, Lewy body dementia, Machado-Joseph disease (Spinocerebellar ataxia type 3), Multiple sclerosis, Multiple System Atrophy, Neuroborreliosis, Parkinson disease, Pelizaeus-Merzbacher Disease, Pick's disease, Primary lateral sclerosis, Prion diseases, Refsum's disease, Sandhoff disease, Schilder's disease, Schizophrenia, Spielmeyer-Vogt-Sjogren-Batten disease (also known as Batten disease), Spinocerebellar ataxia, spinal muscular atrophy, Steele-Richardson-Olszewski disease, Tabes dorsalis. In a preferred embodiment, the neurodegenerative disorder that is diagnosed using the kit of the invention is Alzheimer's disease.

It will be appreciated that the parameter which might be required to decide about a possible disease is not only absolute Aβ40 and Aβ42 concentrations, but also parameters derived from the combination of said values, such as the ratios Aβ40/Aβ42 or Aβ42/Aβ40, the percentages of Aβ42 and Aβ40 over the total Aβ peptides or the addition of the concentrations of Aβ42 and Aβ40.

In a preferred embodiment, the disease that can be diagnosed is Alzheimer's disease, more particularly, sporadic AD, based on the appearance in a sample of an AD patient of lower concentrations of Aβ40 and/or Aβ42 peptides than the amounts of said peptide or peptides in a biological sample from the same origin obtained from a healthy individual.

In use, the kit of the invention allows to carry out a five step method for detecting the amount of a target polypeptide of interest in a sample. Said method, which is another object of the present invention, comprises the steps of
(i) capturing the target polypeptide present in the sample with a first antibody or combination of antibodies which bind specifically said target polypeptide,
(ii) contacting the immune complexes formed in step (a) with a second antibody or combination of antibodies which recognise a different region of the target polypeptide than the first antibody or combination of antibodies,
(iii) contacting the complexes formed in step (ii) with a reagent which shows affinity for the second antibody and which is coupled to a first member of a binding pair,
(iv) contacting the complexes formed in step (iii) with a second member of a binding pair which is coupled to a detectable tag and
(v) detecting the activity of the enzyme or the fluorescent emission of the compound attached to the second member of the binding pair.

In a preferred embodiment, the peptide to be detected is a peptide selected from the group of Aβ40, Aβ42 and a mixture thereof. In a more preferred embodiment, the peptides which are detected correspond to non-oligomeric forms of said peptide, more preferably, monomeric forms of either Aβ40 and Aβ42.

The reagents used in each steps of the method have been described in detail above.

In the first step of the method according to the present invention, the sample which contains Aβ40 and/or Aβ42 peptides is contacted with a first antibody so as to form a first immune complex.

After the first binding step has been carried out, the complexes can be washed to remove any excess of protein/peptide found in the original sample which did not bind to the capture antibody. Preferred washing buffers that can be used in the context of the present invention include any buffer at a pH close to physiological (e.g. 50 mM Tris-HCl) optionally comprising salts (e.g. 150 mM NaCl) and optionally comprising low concentrations of a detergent (e.g. 0.05% Tween-20).

In a second step, the complexes formed between the capture antibody and the Aβ peptide or peptides in the sample are then contacted with the second antibody so as to form a "sandwich-type" immune complex.

After the second step is carried out, the immune complex can be washed to eliminate unspecifically-bound antibodies using essentially the same buffers and procedures as described before.

In the third step, the method of the invention involves contacting the complexes formed between the captured peptide or peptides and the detection antibody with a reagent which shows affinity for the detection antibody and which is coupled to a first member of a binding pair.

In a fourth step, the method of the invention involves contacting the complexes formed between the antibody-binding reagent and the detection antibody with a second member of a binding pair which is coupled to a detectable tag.

In the fifth step of the method according to the invention, the method involves detecting the detectable tag. In the fifth step of the method according to the invention, the method involves detecting the detectable tag. It will be understood that the detection and/or for quantification of the detectable tag depend on the nature of the tag and are known in the art. When an intact substrate or detectable tag contains a luminescent or dye component, detection can be by visual observation on a UV transiluminator, or by using a UV-based charged coupled device (CCD) camera detection system, a laser-based gel scanner, a xenon-arc-based CCD camera detection system, a Polaroid camera combined with a UV-transiluminator as well as a variety of other devices used for detecting luminescence. When the detectable tag is an enzyme, the fifth step of the method according to the invention involves exposing the immunocomplexes labelled with the tag (e.g., the captured peptide, the detection antibody and the reagent labelled with the detectable tag) to activators, substrates, or amplifying agents of the enzyme used as detectable tag. Well known detectable tags capable of generating a detectable signal include enzyme-labeled antibodies. Exemplary enzymes well known for this purpose include horseradish peroxidase, alkaline phosphatase and glycosidases, including β-galactosidase, β-glucosidase and β-glucuronidase. As an example, a reagent which specifically binds to the detection antibody can be tagged with horseradish peroxidase. Upon formation of a capture moiety-detection antibody-reagent complex, detection can then be performed using any of a wide range of well known substrates for the enzyme used as detectable tags.

In another aspect, the invention relates to a method for the diagnosis of a neurodegenerative disease which comprises measuring the levels of Aβ40 and/or Aβ42 in a sample of the subject suspected of carrying said disease and correlating the concentration of one and/or both peptides in the sample o said subject with respect to the concentration of said peptide or peptides in a sample from a healthy individual with the appearance of the neurodegenerative disorder.

It is preferred to carry out the different steps of the method using in parallel the samples to be determined and a number of samples having increasing and known concentrations of the compound which is to be determined. If Aβ40 and/or Aβ42 are to be determined, a standard curve for each peptide must be prepared using increasing concentrations. The standard curve serves the dual purpose of (i) establishing the concentration range wherein the signal increases linearly with the concentration of the target peptide and (ii) determining the concentration of the peptides in the test sample by interpolation of the signal obtained with the test samples in the curve to obtain concentration values. In view of the high sensitivity of the assay of the invention, preferred concentrations of the test samples are e.g. 3.125; 6.25; 12.5; 25; 50; 100 and 200 pg/mL. It will be appreciated that the concentrations of the samples used for obtaining the standard curve will vary for each test substrate. However, determination of the linear range of the assay can be easily determined by the skilled practitioner by conventional means. In view of the higher amounts of Aβ42 and Aβ40 peptides in CSF samples as well as in serum samples of patients with familiar forms of AD as compared to serum from patients with sporadic AD, it is preferred that, if the kit of the invention is used for determining Aβ peptides in samples from CSF or serum from patients with familiar AD, these be diluted so that the final concentrations of Aβ40/Aβ42 are found within the range falls within the linear side of the assay.

The following examples are provided as illustration and should not be construed as limiting the scope of the invention.

### EXAMPLES

### EXAMPLE 1

### Colorimetric ELISA sandwich with biotin-streptavidin amplification

In order to increase the sensitivity, the signal can be amplified using biotin-streptavidin. The plate was coated using the 6E10 mAb capture antibody which recognises amino acids 1-17 in both the amyloid Aß40 and in the amyloid Aß42 peptide. The coating was carried out at a concentration of 5 µg/ml in 100 mM carbonate/bicarbonate buffer, pH=9.6, overnight at 4°C. The plate was then blocked with 300 µl of a blocking solution (50 mM Tris-HCl, pH 8, 0,2% Tween-20, 0,5% BSA) for 3 h at room temperature with shaking or for 2 h at 37° C. When needed, the plates can be treated, after blocking, with 100 µl of a 50 mM Tris-HCl pH 8 solution containing 20 mg/ml trehalose. The plates were left to evaporate until a white halo characteristic of trehalose appears. The plates so treated could be kept at 4°C covered with aluminium foil and are stable for two years.

The samples of the standard curve were prepared from a 200 pg/ml stock solution of the peptides Aβ40 y Aβ42 on plates coated with the 6E10 mAb and treated with trehalose. From these solutions, serial dilutions 1:2 in SDB were made so as to give concentrations of 200, 100, 50, 25, 12.5, 6.25 and 3.125 pg/ml. 100 µl of each diluted or undiluted sample is added diluted or undiluted in SDB (1/1.000.000) and incubated overnight at 4°C (or for 2h at 37°C).

Detection antibody (a polyclonal antibody prepared against a peptide corresponding to the C-terminal region of the Aβ42 peptide or a polyclonal antibody prepared against a peptide corresponding to the C-terminal region of the Aβ40 peptide, depending on whether Aβ42 or Aβ40 is to be detected) was added diluted in SDB. 100 µl are added to each well and were then incubated for 1 h at room temperature.

Next, 100 µl of a 1/5000 dilution in SDB of a biotin-labelled anti-rabbit IgG antibody (SIGMA) were then added and incubated for 1h at room temperature with shaking. Then 100 µl of a 1/4000 dilution in SDB of HRP-coupled Streptavidin (from SIGMA) were added to each well and incubated for 1 h at room temperature.

For developing the plate, 100 µl of the chromogenic substrate TMB (ZEU Inmunotec). TMB was added and incubated in the dark during 15-30 minutes. As stop solution, 50 µl of 1N H2SO4 were added per well. The absorbance at 450 nm was read in a plate reader Synergy HT (BioTek Instruments).

Between each of the steps, the plate was washed using an automatic plate washer (E1x50 Bio Tek Instruments) programmed for performing 5 rinses each time. The washing solution contained 50 mM de Tris-HCl pH 8, 0,05% Tween-20 and 150 mM NaCl (filtered before use).

### EXAMPLE 2

### Fluorescent ELISA Sandwich Assay

The plate was coated with 6E10 in bicarbonate buffer (5 µg/ml) overnight at 4°C. The plate was then blocked 3h at room temperature with shaking (300 (µl/well). The test and standard curve samples were then added to the plates and incubated overnight at 4°C. A 1/4000 dilution of the detection antibody (anti-Aβ40 o anti-Aβ42 serum) was added to each well and incubated for 1h at room temperature with shaking. Serial dilutions of the FITC-coupled anti-antibody (dilutions 1/1000, 1/5000, 1/10000) were added and incubated for 1h at room temperature in the dark. The fluorescence was using an excitation wavelength of 485 nm and an emission wavelength of 528.

Alternatively, the assay is carried out using the *Quanta-Blu* (PIERCE) fluorescent substrate, which increases the sensitivity of the ELISA assay. Maximal excitation is 325 nm and maximal emission is 420 nm. It can be detected in the excitation range of 315-340 nm and 370-470 nm emission range. The QuantaBlu Working Solution is prepared by mixing 9 parts of QuantaBlu Substrate Solution with 1 part of QuantaBlu Stable Peroxidase Solution (solution stable for 24 h at room temperature). It can be incubated from 1,5 minutes to 90 minutes at room temperature and can be read stopping the reaction or without stopping (a blue colour is produced).

The plate is coated with 6E10 mAb in bicarbonate buffer (5 µg/ml) overnight at 4°C and then blocked for 3h at room temperature with shaking (300 µl/well). Different standard curves then prepared with the following concentrations of Aβ42 and Aβ40 peptides:
- 1000, 500, 250, 125, 62.5, 31, 25 and 15.65 pg/mL
- 200, 100, 50, 25, 12.5, 6.25 and 3.125 pg/mL
- 25, 12.5, 6.25, 3.125, 1.56, 0.78 and 0.39 pg/mL
- 10, 5, 2.5, 1.25, 0.625, 0.3125 and 0.156 pg/mL
- 5, 2.5, 1.25, 0.625, 0.3125, 0.156 and 0.078 pg/mL
- 1, 0.5, 0.25, 0.125, 0.0625, 0.03125 and 0.0156 pg/mL

The detection antibody (anti-Aβ40 o anti-Aβ42 serum) is added (diluted at 1/4000) for 1h at room temperature with shaking. The HRP-coupled anti-rabbit IgG 1/1000 is then added and incubated for 1h at room temperature with shaking. For developing the reaction, 100µl of Quanta-Blue Working Solution and then incubated for 30', 60' and 90' at room temperature in the darkness. The fluorescence is then read (Excitation: 360/40 nm; Emission: 460/40 nm) at 30', 60' and 90' without stopping the reaction or stopping the reaction with STOP solution.

### EXAMPLE 3

### Preparation of Aβ40 and Aβ42 standard curves

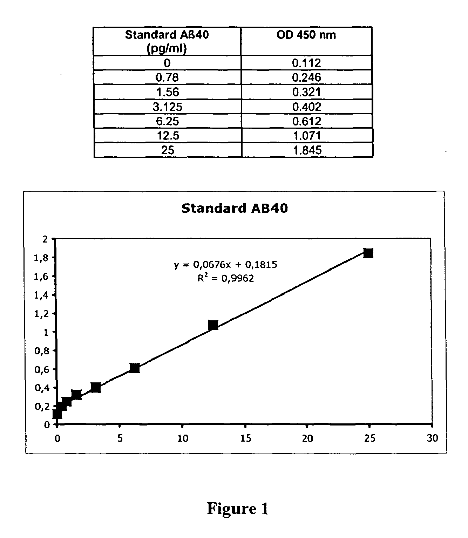

For the preparation of the Aβ40 standard curve, a lyophilised sample of human Aβ40 was reconstituted to 10 µg/mL. From the stock solution, the samples were prepared containing the following concentrations (in pg/mL): 25,000 pg/ml, 2,500 pg/ml, 25 pg/ml, 12.5pg/ml, 6.25 pg/ml, 3.125 pg/ml, 1.56 pg/ml, 0.78 pg/ml. The samples were prepared in the presence of 1 mM of the protease inhibitor AEBSF. The samples were then processed according to the method defined in the previous examples. The results are shown in figure 1.

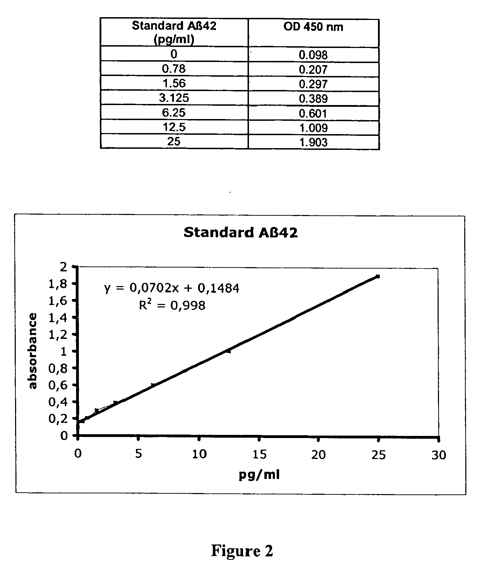

For the preparation of the Aβ42 standard curve, a lyophilised sample of human Aβ42 was_ reconstituted to 10 µg/mL. From the stock solution, samples were prepared containing the following concentrations (in pg/mL): 25,000 pg/ml, 2,500 pg/ml, 25 pg/ml, 12.5pg/ml, 6.25 pg/ml, 3.125 pg/ml, 1.56 pg/ml, 0.78 pg/ml. The samples were prepared in the presence of 1 mM of the protease inhibitor AEBSF. The samples were then processed according to the method defined in the previous examples. The results are shown in figure 2.

### EXAMPLE 4

### Correlation between AD diagnosis and Aβ40/Aβ42 levels

Aβ40 and Aβ42 levels were determined using the ELISA sandwich assay described in the previous examples in plasma samples from a cohort of control subjects and from a cohort of patients diagnosed with AD using the minimental state examination (MMSE) score with a cut-off value of 24. The concentrations in pg/mL of Aβ40 and Aβ42 are shown in Table 1.

**Table 1**

| Healthy | AB40 | AB42 | AB40/AB42 | AB42/AB40 | AB40+AB42 | % AB40 | % AB42 |
|---|---|---|---|---|---|---|---|
| LSA | 31.2 | 160.1 | 0.19 | 5.13 | 191.30 | 16.31 | 83.69 |
| CPB | 93.4 | 159.4 | 0.59 | 1.71 | 252.80 | 36.95 | 63.05 |
| CFA | 730.5 | 893.6 | 0.82 | 1.22 | 1624.10 | 44.98 | 55.02 |
| VCL | 145.0 | 329.6 | 0.44 | 2.27 | 474.60 | 30.55 | 69.45 |
| FLA | 430.1 | 19.6 | 21.94 | 0.05 | 449.70 | 95.64 | 4.36 |
| ILS-7 | 102.9 | 34.2 | 3.01 | 0.33 | 137.05 | 75.08 | 24.92 |
| MPG-8 | 57.0 | 59.2 | 0.96 | 1.04 | 116.17 | 49.07 | 50.93 |
| PLA-17 | 29.6 | 8.7 | 3.39 | 0.30 | 38.27 | 77.21 | 22.79 |
| ARL-24 | 34.0 | 25.8 | 1.32 | 0.76 | 59.82 | 56.89 | 43.11 |
| BGM-28 | 23.4 | 7.4 | 3.16 | 0.32 | 30.77 | 75.95 | 24.05 |
| | | | | | | | |

| AD | AB40 | AB42 | AB40/AB42 | AB42/AB40 | AB40+AB42 | % AB40 | % AB42 |
|---|---|---|---|---|---|---|---|
| BEG | 12.0 | 71.5 | 0.17 | 5.96 | 83.50 | 14.37 | 85.63 |
| CPG | 74.3 | 136.7 | 0.54 | 1.84 | 211.00 | 35.21 | 64.79 |
| VAC | 26.7 | 34.7 | 0.77 | 1.30 | 61.40 | 43.49 | 56.51 |
| MTF | 55.6 | 141.7 | 0.39 | 2.55 | 197.30 | 28.18 | 71.82 |
| CPG | 34.2 | 28.0 | 1.22 | 0.82 | 62.24 | 54.95 | 45.05 |
| MVA-5 | 17.2 | 21.9 | 0.79 | 1.27 | 39.05 | 43.99 | 56.01 |
| 3GS-40 | 13.6 | 16.6 | 0.82 | 1.22 | 30.18 | 45.10 | 54.90 |
| PTG-35 | 37.3 | 24.8 | 1.50 | 0.66 | 62.11 | 60.07 | 39.93 |
| CBC-30 | 11.6 | 6.1 | 1.90 | 0.53 | 17.63 | 65.51 | 34.49 |
| JHC-2 | 25.4 | 23.4 | 1.09 | 0.92 | 48.84 | 52.07 | 47.93 |

Average values were calculated and the results are given in Table 2

**Table 2**

| | Healthy | AD patients |
|---|---|---|
| Aβ40 (pg/mL) | 167,70 | 30,78 |
| Aβ42 (pg/mL) | 169,75 | 50,53 |

## Claims

1. A kit for the detection of a target polypeptide comprising
(i) a first antibody or combination of antibodies which recognise said target polypeptide
(ii) a second antibody or combination of antibodies which recognise a different region of the target polypeptide than the region recognised by the first antibody or combination of antibodies
(iii) a reagent showing affinity for the second antibody which is coupled to a first member of a binding pair and
(iv) a second member of a binding pair coupled to a detectable tag.

2. A kit as defined in claim 1 wherein the first antibody specifically recognises Aβ42 and/or Aβ40 peptides.

3. A kit as defined in claim 2 wherein the first antibody or combination of antibodies recognise a region in Aβ40 and/or Aβ42 which is different from the C-terminal region.

4. A kit as defined in claim 2 wherein the first antibody recognises the N-terminal region of the Aβ42 and Aβ40 peptides.

5. A kit as defined in claim 4 wherein the first antibody is directed against an epitope located within amino acids 1 to 16 of Aβ40 and Aβ42.

6. A kit as defined in claim 1 to 4 wherein the first antibody is a monoclonal antibody.

7. A kit as defined in claim 5 wherein the monoclonal antibody is 6E10 mAb.

8. A kit as defined in any of claims 2 to 7 wherein the second antibody is an antibody selected from the group of
(i) a polyclonal antibody prepared against a peptide corresponding to the C-terminal region of the Aβ42 peptide which binds specifically to Aβ42 without giving any substantial cross-reaction with Aβ40
(ii) a polyclonal antibody prepared against a peptide corresponding to the C-terminal region of the Aβ40 peptide which binds specifically to Aβ40 without giving any substantial cross-reaction with Aβ42 and
(iii) an antibody that recognises simultaneously the C-terminal region of both Aβ40 and Aβ42 and
(iv) a combination of the antibodies under (i) and (ii).

9. A kit as defined in claim 8 wherein the C-terminal region of the Aβ42 peptide used for preparing the second antibody is the peptide of SEQ ID NO:1 or SEQ ID NO:2

10. A kit as defined in claim 8 wherein the C-terminal region of the Aβ40 peptide used for preparing the second antibody is the peptide of SEQ ID NO:3.

11. A kit as defined in any of claims 1 to 10 wherein the first and/or second antibodies or combination of antibodies have been affinity-purified using a polypeptide which comprises the polypeptide used for their preparation.

12. A kit as defined in any of claims 1 to 11 wherein the reagent showing affinity for the second antibody is selected from the group of an anti-IgG antibody, protein A or protein G or a functionally equivalent variant thereof.

13. A kit as defined in any of claims 1 to 12 wherein said first member of a binding pair is biotin.

14. A kit as defined in claim 13 wherein the second member of a binding pair is avidin, streptavidin or a functionally equivalent variant thereof.

15. A kit as defined in claim 1 to 14 wherein said detectable tag is selected from the group of
(i) an enzyme
(ii) a fluorescent molecule

16. A kit as defined in any of claims 1 to 14 further comprising a solid support.

17. A kit as defined in claim 16 wherein one of the antibodies or combination of antibodies are prebound to the solid support.

18. A kit as defined in claim 17 wherein the first antibody is prebound to the solid support.

19. A kit as defined in claim 18 which has been treated with a concentrated solution of trehalose and allowed to dry.

20. A kit as defined in any of claims 2 to 19 further comprising a sample containing Aβ40 and/or Aβ42 peptides.

21. A kit as defined in claim 15 wherein, if the detectable tag is an enzyme, then the kit further comprises a substrate which can be converted by said enzyme into a detectable product.

22. Use of a kit as defined in any of claims 1 to 21 to determine or detect a polypeptide in a sample.

23. Use according to claim 22 wherein the target polypeptide to be determined or detected is selected from the group of Aβ40, Aβ42 or a combination thereof.

24. Use according to claim 23 wherein the sample is selected from the group of blood, plasma, serum, or CSF.

25. Use of a kit according to claims 1 to 24 for the diagnosis of a degenerative disorder in a subject.

26. Use as defined in claim 25 wherein the degenerative disorder is a neurodegenerative disorder.

27. Use as defined in-claim 26 wherein the neurodegenerative disorder is Alzheimer disease.

28. Method for determining or detecting the amount of a target polypeptide in a sample comprising the steps of
(i) capturing the target polypeptide present in the sample with a first antibody or combination of antibodies which bind specifically said target polypeptide,
(ii) contacting the immune complexes formed in step (a) with a second antibody or combination of antibodies which recognise a region of the target polypeptide which is different from the region that is recognised by the first antibody or combination of antibodies,
(iii) contacting the complexes formed in step (ii) with a reagent which shows affinity for the second antibody and which is coupled to a first member of a binding pair,
(iv) contacting the complexes formed in step (iii) with a second member of a binding pair which is coupled to a detectable tag and
(v) detecting or determining the activity or amount of detectable tag attached to the second member of the binding pair.

29. A method as defined in claim 28 wherein the target polypeptide is selected from the group of Aβ42, Aβ40 and the combination of both and both the first antibody or combination of antibodies and the second antibody or combination of antibodies recognise Aβ42 and/or Aβ40.

30. A method as defined in claim 29 wherein the first antibody recognises a region common to Aβ40 and Aβ42 which is different from the C-terminal region.

31. Method as defined in claim 30 wherein the first antibody recognises the N-terminal region of the Aβ42 and Aβ40 peptides.

32. A method as defined in any of claims 29 to 31 wherein the first antibody is directed against an epitope located within amino acids 1 to 16 of Aβ40 and Aβ40.

33. Method as defined in any of claims 28 to 32 wherein the first antibody is a monoclonal antibody.

34. Method as defined in claim 33 wherein the monoclonal capture antibody is the 6E10 mAb.

35. A method as defined in any of claims 29 to 34 wherein the second antibody is an antibody selected from the group of
(i) a polyclonal antibody prepared against a peptide corresponding to the C-terminal region of the Aβ42 peptide which binds specifically to Aβ42 without giving any substantial cross-reaction with Aβ40
(ii) a polyclonal antibody prepared against a peptide corresponding to the C-terminal region of the Aβ40 peptide which binds specifically to Aβ40 without giving any substantial cross-reaction with Aβ42
(iii) an antibody that recognises simultaneously the C-terminal region of both Aβ40 and Aβ42 and
(iv) a combination of the antibodies under (i) and (ii).

36. A method as defined in claim 35 wherein the C-terminal region of the Aβ42 peptide used for preparing the second antibody is the peptide selected from the group of SEQ ID NO:1, SEQ ID NO:2.

37. A method as defined in claim 35 wherein the C-terminal region of the Aβ40 peptide used for preparing the second antibody is the peptide selected from the group of or SEQ ID NO:3.

38. A method as defined in any of claims 28 to 37 wherein the first and/or second antibodies have been affinity-purified using a polypeptide which comprises the sequence of the polypeptide used for their preparation.

39. A method as defined in any of claims 28 to 38 wherein the reagent showing affinity for the second antibody is selected from the group of an anti-IgG antibody, protein A or protein G or a functionally equivalent variant thereof.

40. A method as defined in any of claims 28 to 39 wherein said first member of a binding pair is biotin.

41. A method as defined in claims 40 wherein the second member of a binding pair is avidin, streptavidin or a functionally equivalent variant thereof.

42. A method as defined in claims 28 to 41 wherein said detectable tag is selected from the group of
(i) an enzyme
(ii) a fluorescent molecule

43. A method as defined in any of claims 28 to 42 wherein the biological sample is selected from the group of blood, serum, plasma and CSF.

44. Method as defined in any of claims 28 to 43 wherein the first antibody has been previously immobilised in a solid support.

45. Method for the diagnosis of a degenerative disorder in a subject which comprises determining the amount of Aβ40 or Aβ42 in a sample of a patient using a method as defined in claims 29 to 44 and correlating the concentration of one or both peptides in the sample of said subject with respect to the concentration of said peptide or peptides in a sample from a healthy individual with the appearance of the degenerative disorder.

46. Method according to claim 45 wherein the degenerative disorder is a neurodegenerative disorder.

47. Method as defined in claim 46 wherein the neurodegenerative disorder is Alzheimer disease and wherein if the amount Aβ40 and/or Aβ42 peptides in said samples is lower than the amounts of said peptide or peptides in a biological sample from the same origin obtained from a healthy individual, it is indicative that the subject is suffering from Alzheimer's disease.

48. Method as defined in any of claims 44 to 47 wherein the sample wherein the Aβ40 and/or Aβ42 is to be detected is a plasma or serum sample.


SEQUENCE LISTING

<110>  ARACLON BIOTECH S.L.

<120>  HIGH SENSITIVTY IMMUNOASSAYS AND KITS FOR THE DETERMINATION OF
       PEPTIDES AND PROTEINS OF BIOLOGICAL INTEREST

<130>  P3158EP00

<160>  6

<170>  PatentIn version 3.4

<210>  1
<211>  10
<212>  PRT
<213>  Artificial sequence

<220>
<223>  Peptide used to prepare the anti-Abeta42-specific polyclonal
       antibody

<400>  1

Gly Leu Met Val Gly Gly Val Val Ile Ala
1               5                   10


<210>  2
<211>  8
<212>  PRT
<213>  Artificial sequence

<220>
<223>  Peptide used to prepare the anti-Abeta42-specific polyclonal
       antibody

<400>  2

Met Val Gly Gly Val Val Ile Ala
1               5


<210>  3
<211>  8
<212>  PRT
<213>  Artificial sequence

<220>
<223>  Peptide used to prepare the anti-Abeta40-specific polyclonal
       antibody

<400>  3

Gly Leu Met Val Gly Gly Val Val
1               5


<210>  4
<211>  42
<212>  PRT

<213>  Homo sapiens

<400>  4

<210>  5
<211>  40
<212>  PRT
<213>  Homo sapiens

<400>  5

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  6
<211>  770
<212>  PRT
<213>  Homo sapiens

<400>  6